# EUROPÄISCHE PATENTSCHRIFT

(11) **EP 2 483 663 B1**
(45) Veröffentlichungstag und Bekanntmachung des Hinweises auf die Patenterteilung: **21.10.2015**
(21) Anmeldenummer: 10743137.1
(22) Anmeldetag: 17.08.2010
(51) Int. Cl.: G01N 15/10, B03C 1/28, G01R 33/09, B01L 3/00, G01N 33/543, B03C 1/01, B82Y 25/00, G01N 35/00

(54) **DURCHFLUSSKAMMER MIT ZELLLEITEINRICHTUNG**
FLOW CHAMBER HAVING A CELL GUIDING DEVICE
CHAMBRE D'ÉCOULEMENT ÉQUIPÉE D'UN DISPOSITIF DE GUIDAGE DE CELLULES

(30) Priorität: 30.09.2009 DE 102009047801
(43) Veröffentlichungstag der Anmeldung: 08.08.2012
(73) Patentinhaber: Siemens Aktiengesellschaft, 80333 München (DE)
(72) Erfinder: BÄR, Ludwig, 91056 Erlangen (DE); HAYDEN, Oliver, 91074 Herzogenaurach (DE); ECKERT, Helmut, 91341 Röttenbach (DE); TEDDE, Sandro Francesco, 91058 Erlangen (DE); VIETH, Michael, 91096 Möhrendorf (DE); WEISS, Roland, 91058 Erlangen (DE)
(86) Internationale Anmeldenummer: PCT/EP2010/061931
(87) Internationale Veröffentlichungsnummer: WO 2011/038982

(56) Entgegenhaltungen:
- WO-A1-2009/068598
- WO-A2-02/42734
- WO-A2-2008/048616
- US-A- 5 968 820
- US-A- 5 993 665
- US-A1- 2004 004 043
- US-A1- 2009 001 024
- D. W. INGLIS ET AL.: "Continuous microfluidic immunomagnetic cell separation", APPLIED PHYSICS LETTERS, Bd. 85, Nr. 21, 22. November 2004 (2004-11-22), Seiten 5093-5095, XP002612323,
- YI C ET AL: "Microfluidics technology for manipulation and analysis of biological cells", ANALYTICA CHIMICA ACTA, ELSEVIER, AMSTERDAM, NL, Bd. 560, Nr. 1-2, 23. Februar 2006 (2006-02-23), Seiten 1-23, XP025047235, ISSN: 0003-2670, DOI: DOI:10.1016/J.ACA.2005.12.037 [gefunden am 2006-02-23]
- LIU CHENGXUN ET AL: "Cell manipulation with magnetic particles toward microfluidic cytometry", JOURNAL OF APPLIED PHYSICS, AMERICAN INSTITUTE OF PHYSICS. NEW YORK, US, Bd. 105, Nr. 10, 19. Mai 2009 (2009-05-19) , Seiten 102014-102014, XP012125274, ISSN: 0021-8979, DOI: DOI:10.1063/1.3116091

## Beschreibung

Die Erfindung betrifft eine Durchflusskammer eines Durchflusszytometers, in der markierte Zellen mit Hilfe eines entsprechenden Sensors mit hoher Wahrscheinlichkeit detektierbar sind.

Für ein magnetisches Durchflusszytometer müssen markierte Zellen, die mit Hilfe entsprechender Sensoren detektiert werden sollen, in einer Durchflusskammer oberflächennah über den Sensor transportiert werden. Bspw. kommen zu diesem Zweck GMR-Sensoren (Giant Magneto Resistance bzw. Riesenmagnetowiderstand) oder optische Fluoreszenz- oder Streulicht-Sensor zum Einsatz. Die Nähe der Zelle zum Sensor ist notwendig, da bspw. im Falle eines GMR-Sensors das magnetische Streufeld der magnetischen Marker, das letztlich zur Detektion durch den GMR-Sensor verwendet wird, mit der dritten Potenz zum Abstand vom Sensor abfällt. Entsprechendes gilt für optische Messverfahren.

Um sicherzustellen, dass eine markierte Zelle den Sensor in unmittelbarer Nähe passiert, ist es grundsätzlich denkbar, den Durchmesser des Kanals, durch den das die markierten Zellen aufweisende Medium strömt, möglichst klein zu gestalten, d.h. im Extremfall ist der Kanaldurchmesser gerade so groß, dass einzelne Zellen passieren können. Problematisch wirkt sich hierbei natürlich aus, dass die Anwesenheit von Verunreinigungen bzw. störenden Partikeln sehr schnell zu einer Verstopfung des Kanals führen. Wird der Kanal dagegen größer ausgelegt, steigt auch die Wahrscheinlichkeit, dass einige markierte Zellen den Sensor außerhalb von dessen Reichweite passieren und somit nicht detektiert werden. Dem kann dadurch entgegen gewirkt werden, dass eine größere Anzahl von Sensoren vorgesehen wird, was sich jedoch in einer aufwändigeren Elektronik, niederschlägt.

Die US 7179383 offenbart ein magnetisches Durchflusszytometer.

Es ist daher eine Aufgabe der vorliegenden Erfindung, eine Durchflusskammer anzugeben, bei der eine hohe Wahrscheinlichkeit gegeben ist, eine markierte Zelle mit einem Sensor der Durchflusskammer zu detektieren.

Diese Aufgabe wird durch die in den unabhängigen Ansprüchen angegebenen Erfindungen gelöst. Vorteilhafte Ausgestaltungen ergeben sich aus den abhängigen Ansprüchen.

Bei der erfindungsgemäßen Lösung wird eine Durchflusskammer für ein Durchflusszytometer, welche von einem magnetisch markierte Zellen aufweisenden Medium druchströmbar ist und welche zumindest einen an einer inneren Oberfläche der Durchflusskammer positionierten Sensor zur Zelldetektion aufweist, mit einer magnetischen oder magnetisierbaren Zellleiteinrichtung ausgestattet. Dieser ist in Strömungsrichtung gesehen vor dem Sensor positioniert und dort derart angeordnet sowie ausgebildet, dass sie die strömenden, magnetisch markierten Zellen über den Sensor leitet.

Erfindungsgemäß ist die Zellleiteinrichtung an der inneren Oberfläche der Durchflusskammer angeordnet und weist eine Anzahl n mit n≥1 von magnetischen oder magnetisierbaren und im Wesentlichen parallel zur Strömungsrichtung ausgerichteten Flussstreifen auf, wobei
- die Anzahl n der Flussstreifen der Anzahl der Sensoren entspricht,
- jeweils ein Flussstreifen einem Sensor zugeordnet ist und
- eine von einem Flussstreifen geleitete magnetisch markierte Zelle über den zugeordneten Sensor geleitet wird.

In einer ersten Ausführung weist ein Flussstreifen in Strömungsrichtung gesehen eine durchgehend gleichbleibende Breite aufweist.

In einer zweiten Ausführung verjüngt sich ein Flussstreifen in Strömungsrichtung gesehen insbesondere trichterförmig oder halbtrichterförmig.

In einer dritten Ausführung teilt sich ein einzelner, breiter Flussstreifen in Strömungsrichtung gesehen in mehrere schmalere, im Wesentlichen parallele Teilflussstreifen auf, wobei die Anzahl der Teilflussstreifen der Anzahl der Sensoren entspricht.

In einer vierten Ausführung sind die Flussstreifen fischgrätartig angeordnet.

In einer vorteilhaften Ausgestaltung ist ein Teil eines Flussstreifens, insbesondere der in Strömungsrichtung gesehen hintere Teil, in mehrere hintereinander liegenden und voneinander beabstandete Abschnitte unterteilt.

In einer weiterten vorteilhaften Ausgestaltung ist ein Magnet vorgesehen, der derart an der Durchflusskammer angeordnet ist, dass eine in Richtung der inneren Oberfläche orientierte Kraftwirkung auf die.magnetisch markierten Zellen generiert wird.

In einer weiterten vorteilhaften Ausgestaltung ist der Sensor ein GMR-Snesor.

In einer weiteren Ausführungsform der zuvor beschriebenen Erfindung oder alternativ zur vorhergehend beschriebenen Anordnung und Ausbildung der Zellleiteinrichtung ist eine weitere magnetische oder magnetisierbare Zellleiteinrichtung vorgesehen, die in Strömungsrichtung gesehen hinter dem Sensor positioniert ist.

Bei einem erfindungsgemäßen Verfahren werden zur Detektion magnetisch markierter Zellen eines eine Durchflusskammer eines Durchflusszytometers durchströmenden Mediums mit einem Sensor die strömenden, markierten Zellen mit einer magnetischen oder magnetisierbaren Zellleiteinrichtung, die in Strömungsrichtung gesehen vor dem Sensor positioniert ist, über den Sensor geleitet.

Erfindungsgemäß wird eine weitere Zellleiteinrichtung genutzt, die in Strömungsrichtung gesehen hinter dem Sensor angeordnet ist. Das Medium wird abwechselnd in einer ersten Richtung und in einer zweiten Richtung, die der ersten Richtung entgegengesetzt ist, über den Sensor geleitet.

Vorteile, Merkmale und Einzelheiten der Erfindung ergeben sich aus dem im Folgenden beschriebenen Ausführungsbeispiel sowie anhand der Zeichnungen.

Dabei zeigt:
- Figur 1: eine Durchflusskammer im Querschnitt,
- Figur 2: eine Draufsicht auf eine erste Ausführungsform der Zellleiteinrichtung,
- Figur 3: eine Draufsicht auf eine zweite Ausführungsform der Zellleiteinrichtung,
- Figur 4: eine Draufsicht auf eine dritte Ausführungsform der Zellleiteinrichtung,
- Figur 5: eine Draufsicht auf eine vierte Ausführungsform der Zellleiteinrichtung,
- Figur 6: eine Draufsicht auf eine fünfte Ausführungsform der Zellleiteinrichtung,
- Figur 7: eine Draufsicht auf eine weitere Ausführungsform der Zellleiteinrichtung,
- Figur 8: Draufsichten und eine Seitenansichten dreier Ausführungsformen der Flussstreifen und
- Figur 9: das Prinzip der Zellanreicherung.

In den Figuren sind identische bzw. einander entsprechende Bereiche, Bauteile, Bauteilgruppen oder Verfahrensschritte mit denselben Bezugsziffern gekennzeichnet.

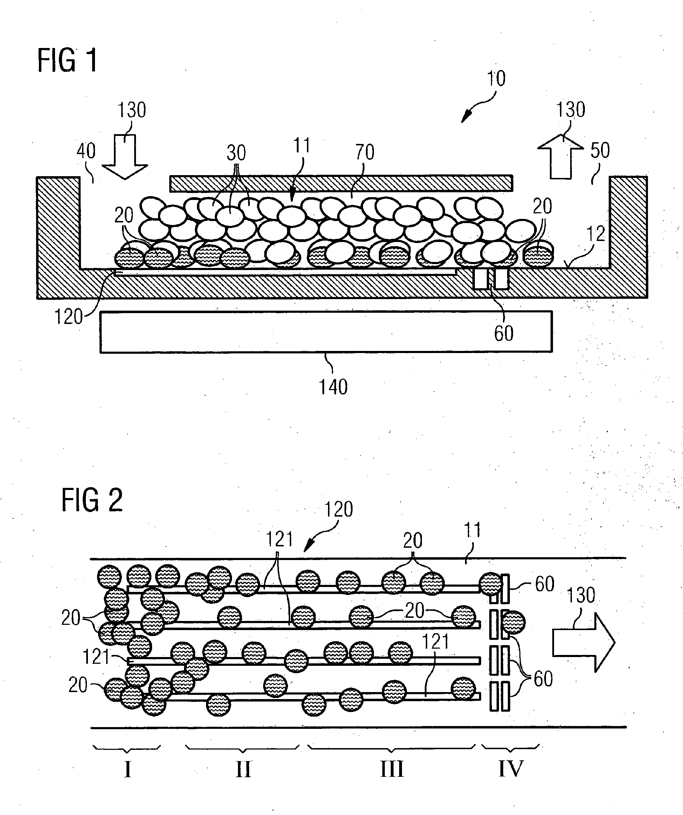

Die Figur 1 zeigt eine Durchflusskammer 10 eines Durchflusszytometers im Querschnitt. Ein Medium 70, das die zu detektierenden, magnetisch markierten Zellen 20 sowie unmarkierte Zellen 30 enthält, gelangt in Strömungsrichtung 130 durch eine Öffnung 40 in die Durchflusskammer 10. Das Medium 70 durchströmt einen Mikrofluidkanal 11 der Kammer 10 und verlässt diesen nach der Detektion wieder durch eine weitere Öffnung 50. Die magnetisch markierten Zellen 20 werden mit Hilfe eines Sensors 60 detektiert. Der Sensor 60 kann bspw. ein GMR-Sensor oder ein optischer Fluoreszenz- oder Streulicht-Sensor sein. Im Folgenden wird exemplarisch von einem GMR-Sensor 60 ausgegangen.

Ebenfalls in der Figur 1 dargestellt ist ein optionaler Permanentmagnet 140, der sich unterhalb des Mikrofluidkanals 11 befindet und der ein magnetisches Feld erzeugt (nicht dargestellt). Dieses Feld wirkt zum Einen anziehend auf die magnetisch markierten Zellen 20, so dass sichergestellt ist, dass sie oberflächennah über den Sensor 60 streifen. Zum Anderen kann der Magnet 140 - speziell im hier angenommenen Fall eines Sensors 60 vom GMR-Typ - verwendet werden, um das zum Betrieb dieses Sensortyps benötigte Gradientenfeld zu erzeugen: Wenn die magnetischen Zellen 20 den GMR-Sensor 60 passieren, beeinflussen sie das am Ort des Sensors herrschende Magnetfeld. Dies wird vom GMR-Sensor registriert und zur Detektion genutzt. Alternativ kann an Stelle des Permanentmagneten 140 natürlich auch eine entsprechende stromdurchflossene Spule verwendet werden. Für den Fall, dass der Sensor 60 ein optischer Fluoreszenz- oder Streulicht-Sensor o.a. ist, wird zum Sensorbetrieb natürlich kein Magnetfeld benötigt. Nichtsdestotrotz kann auch dann ein Magnet vorgesehen sein, um wie erwähnt sicher zu stellen, dass die markierten Zellen 20 den Sensor 60 oberflächennah passieren.

Bei der Dimensionierung des Magneten 140 ist darauf zu achten, dass die Stärke des Magnetfeldes auf die Strömungsgeschwindigkeit des Mediums abgestimmt ist. Ist das Magnetfeld und damit die Haltekraft zu stark, so ist nicht auszuschließen, dass die Strömung gestört wird, da einzelne Zellen 20 evtl. fest gehalten werden. Umgekehrt ist bei zu schwachem ,Magnetfeld davon auszugehen, dass einige der markierten Zellen 20 den Sensor 60 nicht innerhalb von dessen Reichweite passieren, dass sie also nicht detektiert werden.

Mit dem Wechselspiel zwischen der Stärke des Magnetfeldes des Magneten 140 und der bspw. durch (nicht dargestellte) Pumpen erzeugten Strömung 130 bzw. deren Geschwindigkeit kann die Haltekraft für magnetisch markierte Zellen 20 gezielt eingestellt werden, um zum Einen Zellen mit geringer markierungsdichte, d.h. sog. "falsch positive" Zellen, zu entfernen und zum Anderen nur Zellen mit ausreichend starker immunomagnetischer Markierung zum Sensor 60 zu transportieren, wobei ungebundene Marker, bspw. superparamagnetische Partikel, aufgrund der kleineren Haltekraft nicht zum Sensor transportiert werden.

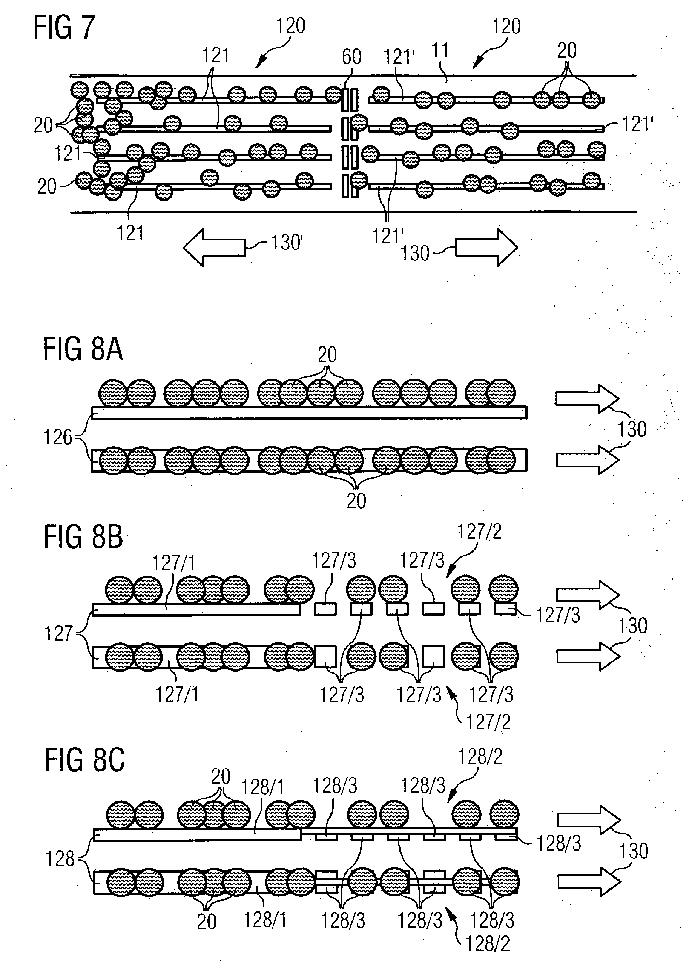

In einer in der Figur 1 nicht dargestellten Anreicherungsvorrichtung, die in der Figur 8 näher beschrieben wird, kann vor der eigentlichen Detektion zunächst eine Anreicherung des Mediums 70 stattfinden, d.h. das die Anreicherungsvorrichtung verlassende, angereicherte Medium 70 würde über die Öffnung 40 in die Durchflusskammer 10 gelangen.

Erfindungsgemäß weist die Durchflusskammer 10 eine Zellleit-einrichtung 120 auf. Diese Einrichtung 120 bewirkt, dass die am Eingang 40 der Durchflusskammer 10 noch stochastisch verteilten, magnetisch markierten Zellen 20 (vgl. Figuren 2 bis 6) gezielt über den Sensor 60 geleitet werden können. Dies hat vorteilhafterweise zur Folge, dass eine wesentlich größere Anzahl von Zellen 20 detektiert werden kann, da deutlich weniger Zellen bspw. seitlich am Sensor 60 vorbei strömen. Es ist demnach nicht mehr dem Zufall überlassen, ob eine markierte Zelle 20 in Reichweite des Sensors 60 gelangt und detektiert werden kann.

Zu diesem Zweck werden magnetische oder magnetisierbare Metallbahnen in Strömungsrichtung auf oder in derjenigen inneren Oberfläche 12 der Durchflusskammer 10 angeordnet, an der auch der Sensor 60 angeordnet ist. Wie im Folgenden anhand der Figuren dargestellt wird, können diese Metallbahnen bzw. "Flussstreifen" bspw. eine gleichbleibende Breite besitzen, sich trichter- oder halbtrichterförmig verjüngen, fächerförmig zusammenlaufen oder auch fischgrätartig angeordnet sein. Andere Anordnungen, die ebenfalls bewirken, dass die markierten Zellen 20 über den Sensor 60 geleitet werden, sind natürlich ebenfalls denkbar. Weiterhin können die Flussstreifen kontinuierlich oder aber diskontinuierlich ausgelegt sein. Die diskontinuierliche Auslegung (vgl. Figur 8B, 8C) bewirkt eine Vereinzelung der Zellen 20, d.h. es wird vermieden, dass mehrere Zellen 20 gleichzeitig bzw. unmittelbar hintereinander über den Sensor 60 streichen. Dadurch, dass also nun einzelne Zellen 20 über den Sensor 60 streichen, wird erreicht, dass eine Einzelzellenanalyse effizienter-durchgeführt werden kann.

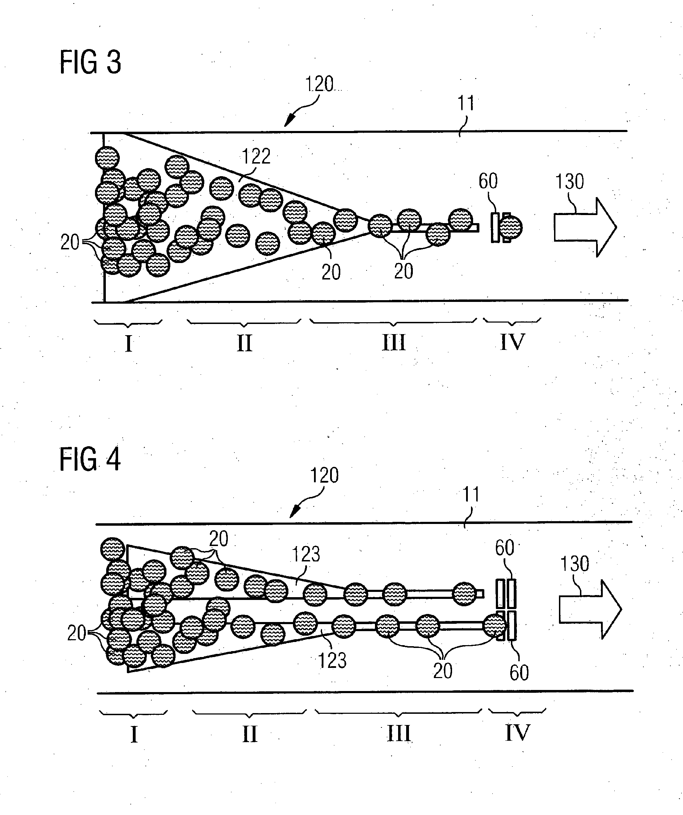

Die Figur 2 zeigt, wie auch die Figuren 3, 4, 5 und 6, eine Draufsicht auf das Innere einer Durchflusskammer 10, wobei der Übersichtlichkeit wegen die unmarkierten Zellen 30 nicht dargestellt sind. Aus dem gleichen Grund sind nur einige wenige der Zellen 20 exemplarisch mit Bezugszeichen versehen. Die Zellleiteinrichtung 120 weist in diesem Äusführungsbeispiel vier Flussstreifen 121 auf, die aus einem magnetischen oder einem magnetisierbaren Material bestehen. Die Flussstreifen 121 sind parallel zueinander angeordnet und in Strömungsrichtung 130 des Mediums ausgerichtet. Die Breite der Flussstreifen 121 kann im Wesentlichen am Durchmesser der Zellen 20 orientiert sein, ist jedoch in der Regel kleiner als die Breite der Sensoren 60.

Durch die Wechselwirkung zwischen den magnetischen Zellen 20 und den magnetischen Flussstreifen 121 wird bewirkt, dass die Zellen 20, während sie mit dem Medium 70 an den Streifen 120 vorbeiströmen, die stochastische Verteilung verlassen und sich an den Streifen 121 anordnen:
- In einem ersten Bereich I sind die Zellen 20 stochastisch verteilt.
- In einem zweiten Bereich II richten sich die Zellen 20 an den Flussstreifen 121 aus.
- In einem dritten Bereich III werden die an den Flussstreifen 121 angeordneten Zellen 20 zu den Sensoren 60 transportiert.
- In einem vierten Bereich IV findet eine (Einzel-) Zellendetektion statt.

Dabei sind die Grenzen der Bereiche I bis IV nicht scharf, sondern in Abhängigkeit bspw. vom Feld des Magneten 140 und von der Strömungsgeschwindigkeit variabel. D.h. die in den Figuren dargestellten Bereiche sind exemplarisch zu verstehen.

Dadurch, dass der magnetische Gradient am Rand des jeweiligen Flussstreifens 121 am größten ist, ist davon auszugehen, das sich die Zellen 20 nicht mittig auf dem jeweiligen Flussstreifen 121 anordnen, sondern an dessen Rand.

In Strömungsrichtung gesehen befindet sich hinter jedem Flussstreifen 121, d.h. in Verlängerung des Streifens 121, ein Sensor 60, so dass die markierten und geordneten Zellen 20 mit Hilfe der Zellleiteinrichtung 120 gezielt über den Sensor 60 geleitet werden können. Abgesehen von wenigen Ausnahmen, die nicht von den magnetischen Flussstreifen 121 erfasst wurden und daher nicht zu den Sensoren 60 geleitet werden, kann davon ausgegangen werden, dass ein Großteil der markierten Zellen 20 des Mediums 70 in Reichweite der Sensoren 60 gelangen, so dass mit der erfindungsgemäßen Anordnung eine wesentlich höhere Ausbeute erzielt werden kann, was sich bspw. bei gleichbleibender Statistik in einer kürzeren Messzeit oder bei gleichbleibender Messzeit in einer verbesserten Statistik äußert.

Die Flussstreifen können bspw. aus Nickel bestehen und ≤ 10µm breit sowie 100-500nm dick sein. Dicken in einer Größenordnung von 1µm sind aber ebenfalls denkbar. Der Mikrofluidkanal 11 ist typischerweise 100-400µm breit, 100µm hoch und etwa 1mm lang. Die GMR-Sensoren 60 sind etwa 25-30µm lang (in einer Richtung senkrecht zur Strömungsrichtung 130).

Die Figur 3 zeigt ein weiteres Ausführungsbeispiel einer Zellleiteinrichtung 120. Hier weist die Zellleiteinrichtung 120 nur einen Flussstreifen 122 auf, der sich jedoch in Strömungsrichtung 130 gesehen trichterförmig verjüngt, bis er schließlich eine Breite aufweist, die in etwa dem Durchmesser der Zellen 20 entspricht. An seiner breiten Seite deckt der Streifen 122 die komplette Breite der Durchflusszelle 10 bzw. des Mikrofluidkanals 11 ab. Dieser breite Bereich des Streifens wirkt quasi als Sammler, mit dem die Zellen 20 auf den schmalen Flussstreifen hin geführt werden können.

Auch in diesem Ausführungsbeispiel besteht der Flussstreifen 122 aus einem magnetischen oder einem magnetisierbaren Material, so dass auch hier,die anfangs stochastisch verteilten, magnetisch markierten Zellen 20 geordnet und schließlich über den Sensor 60 geleitet werden können.

Der Vorteil der Anordnung der Figur 3 gegenüber derjenigen der Figur 2 liegt bspw. darin, dass hier nur ein Sensor 60 benötigt wird. Dies ermöglicht eine Vereinfachung der Ausleseelektronik.

In einem dritten Ausführungsbeispiel der Zellleiteinrichtung 120, das in der Figur 4 dargestellt ist, besteht diese aus zwei magnetischen oder magnetisierbaren Flussstreifen 123, die sich jeweils in Strömungsrichtung 130 gesehen halbtrichterförmig verjüngen. Wie in den anderen Ausführungsbeispielen ist auch hier jedem Flussstreifen 123 ein Sensor 60 zugeordnet, der sich in Strömungsrichtung 130 hinter den Flussstreifen 123 befindet und über den die markierten Zellen 20 geleitet werden.

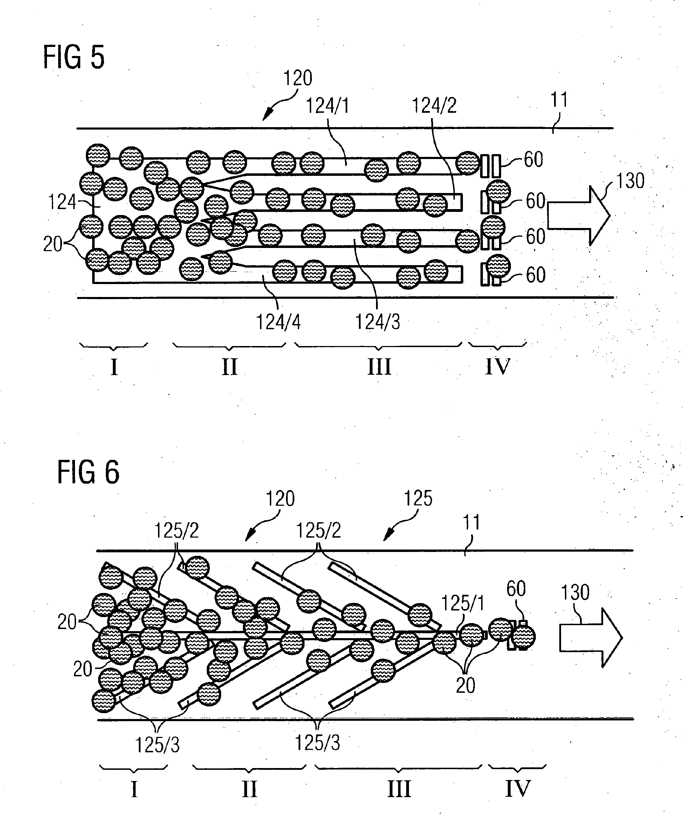

Ein viertes Ausführungsbeispiel ist in der Figur 5 dargestellt. Der hier gezeigte Flussstreifen 124 ist wie in den Beispielen der Figuren 2 und 3 eingangsseitig, d.h. im Bereich I, vergleichsweise breit ausgebildet. Der einzelne, breite Flussstreifen 124 geht jedoch in vier Teilflussstreifen 124/1 bis 124/4 über, über die die Zellen 20 wie in den vorherigen Ausführungsbeispielen zu den Sensoren 60 geleitet werden.

Die Figur 6 zeigt ein fünftes Ausführungsbeispiel der Zellleiteinrichtung 120. Hier sind die Flussstreifen 125 fischgrätförmig angeordnet, d.h. es ist zum Einen ein zentraler Flussstreifen 125/1 vorgesehen, der in seiner Verlängerung zum Sensor 60 führt. Zum Anderen sind weitere Flussstreifen 125/2, 125/3 vorgesehen, die unter einem Winkel von bspw. ±45° zur Strömungsrichtung 130 angeordnet sind, so dass die magnetisch markierten Zellen 20 zunächst zum zentralen Flussstreifen 125/1 und von diesem über den Sensor 60 geleitet werden.

Die Figur 7 zeigt eine Ausführungsform, die in der Anordnung der Flussstreifen 121 prinzipell derjenigen der Figur 2 entspricht. Im Unterschied zur Figur 2 sind hier jedoch in Strömungsrichtung gesehen sowohl vor als auch hinter den Sensoren 60 Flussstreifen 121, 121' angeordnet. Bei einem entsprechenden Detektionsverfahren würden das Medium und damit die markierten Zellen 20 bspw. zur Verbesserung der Statistik abwechselnd in eine erste Strömungsrichtung 130 und in die entgegengesetzte Richtung 130' befördert. Die Zellen 20 streichen demnach mehrfach über die Sensoren 60.

Grundsätzlich kann die Ausführungsform der Figur 7 mit beiderseits der Sensoren 60 angeordneter Zellleiteinrichtung natürlich auch entsprechend den Ausführungsformen der Zellleiteinrichtungen der Figuren 3 bis 6 realisiert werden. Da die den Sensor 60 passierenden Zellen 20 aber in der Regel schon geordnet sind, d.h. nicht mehr stochastisch verteilt, reicht es in der Regel aus, die weitere Zellleiteinrichtung 120' wie in der Figur 7 dargestellt auszubilden. Eine Art "Sammler", wie ihn die Zellleiteinrichtungen 120 insbesondere der Figuren 3, 4 und 5 im Bereich I aufweisen und der in erster Linie dazu dient, die stochastisch verteiliten Zellen 20 zu den Einzelbahnen hin zu leiten, wäre im Falle der weiteren Zellleiteinrichtung nur dann notwendig, wenn es möglich sein soll, der Durchflusskammer 10 sowohl über die Öffnung 40 als auch über die Öffnung 50 ein Medium zuzuführen.

Die Figuren 8A, 8B, 8C zeigen unterschiedliche Ausführungformen der einzelnen Flussstreifen. Dabei zeigen die Figuren jeweils eine Seitenansicht und eine Draufsicht des Flussstreifens mit daran angeordneten magnetisch markierten Zellen 20.

Der Flussstreifen 126 der Figur 8A ist kontinuierlich ausgeführt, so wie auch in den Figuren 1 bis 7 dargestellt.

In der Figur 8B ist dagegen ein diskontinuierlicher Flussstreifen 127 dargestellt. Dieser ist im in Strömungsrichtung. 130 gesehenen vorderen Teil 127/1 ebenfalls kontinuierlich ausgebildet. Der hintere Teil 127/2 des Flussstreifens 127 ist jedoch diskontinuierlich, d.h. der Streifen ist hier in mehrere hintereinander liegende Abschnitte 127/3 unterbrochen. Wie oben beschrieben wirkt sich dies vorteilhaft auf die Möglichkeit der Einzelzellendetektion aus. Die Länge der einzelen Abschnitte 127/3 kann bspw. der Breite des Streifens und/oder in etwa dem Zelldurchmesser entsprechen.

Der Flussstreifen 128 der Figur 8C entspricht im Wesentlichen demjenigen der Figur 8B, d.h. es ist ein vorderer, kontinuierlicher Teil 128/1 und ein hinterer, diskontinuierlicher Teil 128/2 mit einzelnen Abschnitten 128/3 vorgesehen. Zusätzlich ist jedoch auf die Abschnitte 128/3 ein kontinuierlicher Streifen 128/4 aufgebracht, der bspw. verhindert, dass durch evtl. turbulente Anteile in der Strömung Zellen 20 in den Bereichen zwischen den Abschnitten 128/3 abgelenkt werden.

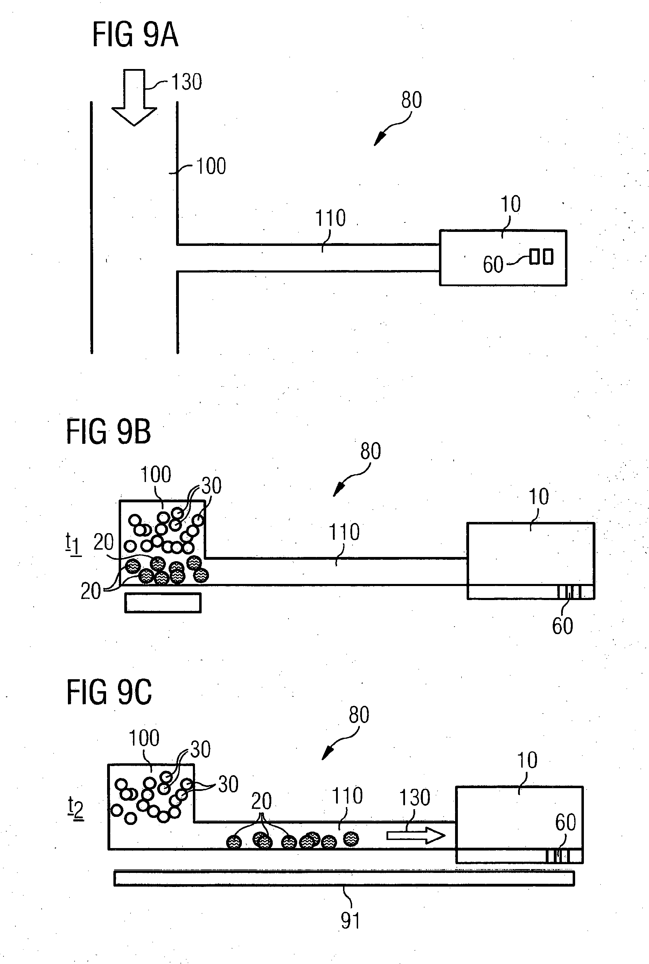

In der Figur 9 ist das Prinzip der Anreicherung vereinfacht dargestellt. Dabei zeigt die Figur 9A eine Draufsicht auf die Anreicherungsvorrichtung 80, während in den Figuren 9B und 9C zwei Seitenansichten bzw. Querschnitte zu aufeinander folgenden Zeitpunkten t1, t2 (t2>t1) der Vorrichtung 80 dargestellt sind. Typischerweise ist die Konzentration der magnetisch markierten Zellen 20 im ursprünglichen Medium, bspw. Vollblut, vergleichsweise gering. Eine Analyse würde zeitlich sehr aufwändig sein. Das ursprüngliche Medium, das über einen Kanal 100 in die Anreicherungsvorrichtung 80 strömt, wird daher vor dem Detektionsschritt angereichert, wobei der Anteil der markierten Zellen 20 im Vergleich zum Anteil unmarkierter Zellen 30 im Medium erhöht werden soll.

In der Figur 9 ist die sog. semikontinuierliche Anreicherung dargestellt, bei der zunächst zum Zeitpunkt t1 (vgl. Figur 9B) der Anreicherungsschritt stattfindet und anschließend zum Zeitpunkt t2 (Figur 9C) das angereicherte Medium zur Durchflusskammer transportiert wird. Daran anschließend würde eine erneute Anreicherung stattfinden (nicht dargestellt) usw.

Zum Zwecke der Anreicherung wird ein Magnet 90 verwendet, der ein erstes magnetisches Feld (nicht dargestellt) in einer Größenordnung von etwa 100-1000 mT generiert. Dies bewirkt, dass die magnetisch markierten Zellen 20 auf die Seite des Kanals 100 gezogen werden, auf der der Magnet 90 angeordnet ist. Dementsprechend ist die Konzentration markierter Zellen 20 an dieser Seite des Kanals 100 deutlich erhöht. An eben dieser Seite ist darüber hinaus ein weiterer Kanal 110 vorgesehen, über den das nun angereicherte Medium zur Durchflusskammer 10 gelangt, die in der Figur 9 nur symbolisiert dargestellt ist. Um die magnetisch markierten Zellen 20 auch im Kanal 110 und schließlich in der Durchflusskammer 10 an derjenigen Seite zu halten, an der auch der Sensor 60 positionier ist, ist ein weiterer Magnet 91 vorgesehen, der jedoch ein schwächeres Magnetfeld erzeugt, als der Magnet 90, bspw. in einer Größenordnung von bis zu 100mT.

Konzeptionell kann das mit der erfindungsgemäßen Durchflusskammer durchführbare Verfahren bspw. für Säugetierzellen, Mikroorganismen oder magnetische Beads eingesetzt werden. Die magnetische Durchflusszytometrie kann in Kombination mit optischen (z.B. Fluoreszenz, Streulicht) oder anderen nicht-magnetischen Detektionsverfahren (z.B. radiochemisch, elektrisch) kombiniert werden, um in-situ Beobachtungen vorzunehmen oder weitere Analysen durchzuführen.

## Patentansprüche

1. Durchflusskammer für ein Durchflusszytometer, welche von einem magnetisch markierte Zellen (20) aufweisenden Medium (70) druchströmbar ist, mit
- zumindest einem an einer inneren Oberfläche (12) der Durchflusskammer (10) positionierten Sensor (60) zur Zelldetektion,
- einer magnetischen oder magnetisierbaren Zellleiteinrichtung (120), die in Strömungsrichtung (130) gesehen vor dem Sensor (60) positioniert ist und die derart angeordnet und ausgebildet ist, dass sie die strömenden, magnetisch markierten Zellen (20) über den Sensor (60) leitet,
wobei die Zellleiteinrichtung (120) an der inneren Oberfläche (12) der Durchflusskammer (10) angeordnet ist und eine Anzahl n mit n≥1 von magnetischen oder magnetisierbaren und im Wesentlichen parallel zur Strömungsrichtung ausgerichteten Flussstreifen (121-128) aufweist, **dadurch gekennzeichnet, dass**
- die Anzahl n der Flussstreifen (121-128) der Anzahl der Sensoren (60) entspricht,
- jeweils ein Flussstreifen (121-128) einem Sensor (60) zugeordnet ist,
- eine von einem Flussstreifen (121-128) geleitete magnetisch markierte Zelle (20) über den zugeordneten Sensor (60) geleitet wird.

2. Durchflusskammer nach Anspruch 1, **dadurch gekennzeichnet, dass** ein Flussstreifen (121) in Strömungsrichtung (130) gesehen eine durchgehend gleichbleibende Breite aufweist.

3. Durchflusskammer nach Anspruch 2, **dadurch gekennzeichnet, dass** sich ein Flussstreifen (122, 123) in Strömungsrichtung (130) gesehen verjüngt, insbesondere trichterförmig oder halbtrichterförmig.

4. Durchflusskammer nach Anspruch 2, **dadurch gekennzeichnet, dass** sich ein einzelner, breiter Flussstreifen (124) in Strömungsrichtung (130) gesehen in mehrere schmalere, im Wesentlichen parallele Teilflussstreifen (124/1-124/4) aufteilt, wobei die Anzahl der Teilflussstreifen (124/1-124/4) der Anzahl der Sensoren (60) entspricht.

5. Durchflusskammer nach Anspruch 2, **dadurch gekennzeichnet, dass** die Flussstreifen (125) fischgrätartig angeordnet sind.

6. Durchflusskammer nach einem der Ansprüche 2 bis 6, **dadurch gekennzeichnet, dass** ein Teil (127-2) eines Flussstreifens (127), insbesondere der in Strömungsrichtung gesehen hintere Teil (127-2), in mehrere hintereinander liegenden und voneinander beabstandete Abschnitte (127-3) unterteilt ist.

7. Durchflusskammer nach einem der vorhergehenden Ansprüche, **dadurch gekennzeichnet, dass** ein Magnet (140) vorgesehen ist, der derart an der Durchflusskammer (10) angeordnet ist, dass eine in Richtung der inneren Oberfläche (12) orientierte Kraftwirkung auf die magnetisch markierten Zellen (20) generiert wird.

8. Durchflusskammer nach einem der vorhergehenden Ansprüche, **dadurch gekennzeichnet, dass** der Sensor (60) ein GMR-Snesor ist.

9. Durchflusskammer für ein Dürchflusszytometer, welche von einem magnetisch markierte Zellen (20) aufweisenden Medium (70) druchströmbar ist, mit
- zumindest einem an einer inneren Oberfläche (12) der Durchflusskammer (10) positionierten Sensor (60) zur Zelldetektion,
- einer magnetischen oder magnetisierbaren Zellleiteinrichtung (120), die in Strömungsrichtung (130) gesehen vor dem Sensor (60) positioniert ist und die derart angeordnet und ausgebildet ist, dass sie die strömenden, magnetisch markierten Zellen (20) über den Sensor (60) leitet, insbesondere nach einem der vorhergehenden Ansprüche, **dadurch gekennzeichnet, dass** eine weitere magnetischen oder magnetisierbaren Zellleiteinrichtung (120') in Strömungsrichtung (130) gesehen hinter dem Sensor (60) positioniert ist.

10. Verfahren zur Detektion magnetisch markierter Zellen (20) eines eine Durchflusskammer (10) eines Durchflusszytometers durchströmenden Mediums (70) mit einem Sensor (60), bei dem die strömenden, markierten Zellen (20) mit einer magnetischen oder magnetisierbaren Zellleiteinrichtung (120), die in Strömungsrichtung (130) gesehen vor dem Sensor (60) positioniert ist, über den Sensor (60) geleitet werden, **dadurch gekennzeichnet, dass** eine weitere Zellleiteinrichtung (120') in Strömungsrichtung (130) gesehen hinter dem Sensor (60) angeordnet ist, wobei das Medium (70) abwechselnd in einer ersten Richtung (130) und in einer zweiten Richtung (130'), die der ersten Richtung entgegengesetzt ist, über den Sensor (60) strömt.

## Claims

1. Flow chamber for a flow cytometer, through which a medium (70) comprising magnetically labelled cells (20) may flow, having
- at least one sensor (60) positioned on an internal surface (12) of the flow chamber (10) for detecting cells,
- a magnetic or magnetisable cell-guiding device (120), which is positioned upstream of the sensor (60) in the direction of flow (130) and is arranged and constructed such that it guides the flowing, magnetically labelled cells (20) over the sensor (60),
wherein the cell-guiding device (120) is arranged on the internal surface (12) of the flow chamber (10) and comprises a number n, with n≥1, of magnetic or magnetisable flow strips (121-128) oriented substantially parallel to the direction of flow, **characterised in that**
- the number n of flow strips (121-128) corresponds to the number of sensors (60),
- one flow strip (121-128) is in each case assigned to one sensor (60),
- a magnetically labelled cell (20) guided by a flow strip (121-128) is guided over the assigned sensor (60).

2. Flow chamber according to claim 1, **characterised in that** a flow strip (121) is of a width which remains constant throughout in the direction of flow (130).

3. Flow chamber according to claim 2, **characterised in that** a flow strip (122, 123) tapers, in particular in the manner of a funnel or half funnel, in the direction of flow (130).

4. Flow chamber according to claim 2, **characterised in that** an individual, wide flow strip (124) is subdivided in the direction of flow (130) into a plurality of substantially parallel flow sub-strips (124/1-124/4), wherein the number of flow sub-strips (124/1-124/4) corresponds to the number of sensors (60).

5. Flow chamber according to claim 2, **characterised in that** the flow strips (125) are arranged in a herringbone pattern.

6. Flow chamber according to one of claims 2 to 6, **characterised in that** part (127-2) of a flow strip (127), in particular the downstream part (127-2) in the direction of flow, is subdivided into a plurality of portions (127-3) lying downstream of one another and spaced apart from one another.

7. Flow chamber according to one of the preceding claims, **characterised in that** a magnet (140) is provided, which is arranged in such a manner on the flow chamber (10) that a force directed towards the internal surface (12) is generated which acts on the magnetically labelled cells (20).

8. Flow chamber according to one of the preceding claims, **characterised in that** the sensor (60) is a GMR sensor.

9. Flow chamber for a flow cytometer, through which a medium (70) comprising magnetically labelled cells (20) may flow, having
- at least one sensor (60) positioned on an internal surface (12) of the flow chamber (10) for detecting cells,
- a magnetic or magnetisable cell-guiding device (120), which is positioned upstream of the sensor (60) in the direction of flow (130) and is arranged and constructed such that it guides the flowing, magnetically labelled cells (20) over the sensor (60) especially according to one of the preceding claims, **characterised in that** a further magnetic or magnetisable cell-guiding device (120') is positioned downstream of the sensor (60) in the direction of flow (130).

10. Method for detecting magnetically labelled cells (20) in a medium (70) flowing through a flow chamber (10) of a flow cytometer with a sensor (60), in which the flowing, labelled cells (20) are guided over the sensor (60) with a magnetic or magnetisable cell-guiding device (120), which is positioned upstream of the sensor (60) in the direction of flow (130), **characterised in that** a further cell-guiding device (120') is arranged downstream of the sensor (60) in the direction of flow (130), wherein the medium (70) flows over the sensor (60) alternately in a first direction (130) and in a second direction (130'), which is contrary to the first direction.

## Revendications

1. Chambre d'écoulement pour un cytomètre en flux, laquelle chambre est traversable par un milieu (70) comportant des cellules magnétiquement marquées (20), comprenant
- au moins un détecteur (60) positionné sur une surface intérieure (12) de la chambre d'écoulement (10) pour la détection de cellules,
- un dispositif de guidage de cellules magnétique ou magnétisable (120) qui, vu dans le sens d'écoulement (130), est positionné en amont du détecteur (60) et qui est disposé et conçu de façon à guider le flux de cellules magnétiquement marquées (20) au-dessus du détecteur (60),
ledit dispositif de guidage de cellules (120) étant placé sur la surface intérieure (12) de la chambre d'écoulement (10) et présentant un nombre n, avec n≥1, de bandes d'écoulement magnétiques ou magnétisables (121-128) orientées sensiblement parallèlement au sens d'écoulement, **caractérisée en ce que**
- le nombre n de bandes d'écoulement (121-128) correspond au nombre de détecteurs (60),
- respectivement une bande d'écoulement (121-128) est associée à un détecteur (60),
- une cellule magnétiquement marquée (20) guidée par une bande d'écoulement (121-128) est guidée au-dessus du détecteur associé (60).

2. Chambre d'écoulement selon la revendication 1, **caractérisée en ce qu'**une bande d'écoulement (121), vue dans le sens d'écoulement (130), présente une largeur constante de bout en bout.

3. Chambre d'écoulement selon la revendication 2, **caractérisée en ce qu'**une bande d'écoulement (122, 123), vue dans le sens d'écoulement (130), se rétrécit, notamment en entonnoir ou en demi-entonnoir.

4. Chambre d'écoulement selon la revendication 2, **caractérisée en ce qu'**une bande d'écoulement individuelle large (124), vue dans le sens d'écoulement (130), se divise en plusieurs bandes d'écoulement partielles plus étroites (124/1-124/4) sensiblement parallèles, le nombre de bandes d'écoulement partielles (124/1-124/4) correspondant au nombre de détecteurs (60).

5. Chambre d'écoulement selon la revendication 2, **caractérisée en ce que** les bandes d'écoulement (125) sont disposées en arête de poisson.

6. Chambre d'écoulement selon l'une des revendications 2 à 6, **caractérisée en ce qu'**une partie (127-2) d'une bande d'écoulement (127), notamment la partie (127-2) postérieure vue dans le sens d'écoulement, est subdivisée en plusieurs portions (127-3) situées les unes derrière les autres et éloignées les unes des autres.

7. Chambre d'écoulement selon l'une des revendications précédentes, **caractérisée en ce qu'**il est prévu un aimant (140), disposé sur la chambre d'écoulement (10) de manière à générer un effet de force orienté vers la surface intérieure (12) sur les cellules magnétiquement marquées (20).

8. Chambre d'écoulement selon l'une des revendications précédentes, **caractérisée en ce que** le détecteur (60) est un détecteur GMR.

9. Chambre d'écoulement pour un cytomètre en flux, laquelle chambre est traversable par un milieu (70) comportant des cellules magnétiquement marquées (20), comprenant
- au moins un détecteur (60) positionné sur une surface intérieure (12) de la chambre d'écoulement (10) pour la détection de cellules,
- un dispositif de guidage de cellules magnétique ou magnétisable (120) qui, vu dans le sens d'écoulement (130), est positionné en amont du détecteur (60) et qui est disposé et conçu de façon à guider le flux de cellules magnétiquement marquées (20) au-dessus du détecteur (60), en particulier selon l'une des revendications précédentes, **caractérisé en ce qu'**un autre dispositif de guidage de cellules magnétique ou magnétisable (120'), vu dans le sens d'écoulement (130), est positionné en aval du détecteur (60).

10. Procédé de détection de cellules magnétiquement marquées (20) d'un milieu (70) traversant une chambre d'écoulement (10) d'un cytomètre en flux au moyen d'un détecteur (60), dans lequel le flux de cellules magnétiquement marquées (20) est guidé au-dessus du détecteur (60) par un dispositif de guidage de cellules magnétique ou magnétisable (120) qui, vu dans le sens d'écoulement (130), est positionné en amont du détecteur (60), **caractérisé en ce qu'**un autre dispositif de guidage de cellules magnétique ou magnétisable (120'), vu dans le sens d'écoulement (130), est positionné en aval du détecteur (60), le milieu (70) passant au-dessus du détecteur (60), alternativement, dans un premier sens (130) et dans un second sens (130') opposé au premier.
